# EUROPÄISCHE PATENTSCHRIFT

(11) **EP 4 125 736 B1**
(45) Veröffentlichungstag und Bekanntmachung des Hinweises auf die Patenterteilung: **12.02.2025**
(21) Anmeldenummer: 21716372.4
(22) Anmeldetag: 31.03.2021
(51) Int. Cl.: A61F 2/50

(54) **VERFAHREN ZUM HERSTELLEN EINES VERBINDUNGSELEMENTES**
METHOD FOR PRODUCING A CONNECTION ELEMENT
PROCÉDÉ PERMETTANT DE PRODUIRE UN ÉLÉMENT DE LIAISON

(30) Priorität: 31.03.2020 DE 102020108877
(43) Veröffentlichungstag der Anmeldung: 08.02.2023
(73) Patentinhaber: Ottobock SE & Co. KGaA, 37115 Duderstadt (DE)
(72) Erfinder: LEINIGER, Andreas, 37327 Leinefeld OT Birkungen (DE); VOLKMAR, Julia, 37115 Duderstadt (DE); PAHL, Erik, 37115 Duderstadt (DE)
(74) Vertreter: Gramm, Lins & Partner Patent- und Rechtsanwälte PartGmbB
(86) Internationale Anmeldenummer: PCT/EP2021/058515
(87) Internationale Veröffentlichungsnummer: WO 2021/198378

(56) Entgegenhaltungen:
- WO-A1-2018/148039
- FR-A1- 3 054 691
- US-B2- 10 779 982

## Beschreibung

Die Erfindung betrifft ein Verfahren zum Herstellen eines Verbindungselementes zum Verbinden zweier Bauteile einer orthopädietechnischen Einrichtung für ein Körperteil. Die Erfindung betrifft zudem ein Verfahren zum Herstellen der orthopädietechnischen Einrichtung.

Orthopädietechnische Einrichtungen sind aus dem Stand der Technik seit langem bekannt und umfassen beispielsweise Orthesen, die ein vorhandenes Körperteil des Trägers schützen, stützen oder beispielsweise in der Bewegung einschränken können, Prothesen, die ein Körperteil des Trägers ersetzen und Exoskelette zur Unterstützung von Körperteilen.

Eine Orthese für ein Körperteil wird in der Regel an diesem Körperteil angeordnet. Eine Knieorthese wird beispielsweise an einem Knie des Trägers und eine Ellenbogenorthese an einem Ellenbogen des Trägers angeordnet. Im Sinne der vorliegenden Anmeldung ist eine solche Orthese für das jeweilige Körperteil ausgebildet. Eine Prothese hingegen wird an einem vorhandenen Körperteil des Trägers angeordnet. Eine Knieorthese kann beispielsweise mittels eines Prothesenschaftes an einem Oberschenkelstumpf angeordnet werden. Im Sinne der vorliegenden Erfindung ist eine solche Knieprothese eine orthopädietechnische Einrichtung für den Oberschenkel. Eine orthopädietechnische Einrichtung ist im Sinne der vorliegenden Erfindung eine orthopädietechnische Einrichtung für ein Körperteil, wenn sie an diesem Körperteil angeordnet wird oder dieses Körperteil unterstützt, schützt, stützt oder überspannt. Eine Knieorthese wird in der Regel zwar am Oberschenkel und dem Unterschenkel angeordnet, überspannt und stützt jedoch das Knie. Sie kann also sowohl als eine orthopädietechnische Einrichtung für das Knie als auch für den Oberschenkel und den Unterschenkel angesehen und bezeichnet werden.

Eine orthopädietechnische Einrichtung besteht in der Regel aus mehr als zwei Bauteilen, die miteinander verbunden werden müssen. Dazu werden Verbindungselemente verwendet. Diese können zwei Bauteile starr miteinander verbinden, so dass im verbundenen Zustand keine Bewegung der beiden Bauteile relativ zueinander möglich ist. Dies ist beispielsweise bei einem Schaftadapter der Fall, der an einem Prothesenschaft angeordnet wird und diesen mit einem weiteren Prothesenelement, beispielsweise einem Prothesenknie oder einem Rohrelement verbindet. Die Orientierung der beiden Bauteile relativ zueinander ist gegebenenfalls einstellbar, im verbundenen Zustand ist eine Relativbewegung jedoch unerwünscht und deswegen ausgeschlossen.

Ein Verbindungselement kann aber auch derart ausgebildet sein, dass die beiden Bauteile im verbundenen Zustand relativ zueinander bewegbar sind. Dies ist beispielsweise der Fall, wenn das Verbindungselement ein Gelenk aufweist oder als Gelenk ausgebildet ist. Ein solches Gelenk verbindet beispielsweise ein Oberschenkelelement und ein Unterschenkelelement einer Knieorthese oder ein Unterschenkelelement und ein Fußelement einer Knöchel-orthese.

Aus der WO 2018/148039 A1 und der US 10,779,982 B2 sind jeweils Verfahren bekannt, bei denen einzelne Bauteile einer orthopädietechnischen Einrichtung anhand von Scan-Daten eines Körperteils hergestellt und so besonders gut und individuell an dieses Körperteil angepasst sind. Eine Verbindung dieser Bauteile mittels eines gesonderten Verbindungselementes ist nicht beschrieben.

Insbesondere für den Fall, dass das Verbindungselement Bauteile miteinander verbindet, von denen wenigstens eines, bevorzugt jedoch beide, individuell für den Träger der orthopädietechnischen Einrichtung angefertigt wurden, etwa als Kohlefaserverbund-Bauteil, wird oft auch das Verbindungselement an die individuellen Bedürfnisse und Gegebenheiten angepasst. So ist beispielsweise ein Gelenkschenkel eines derartigen Verbindungselementes als Metallbauteil ausgebildet, das eine eben Fläche bildet und eine Länge aufweist, die eine ausreichende Verbindung mit einem der zu verbindenden Bauteile ermöglicht. Dieser Gelenkschenkel wird beim Herstellen der orthopädietechnischen Einrichtung verformt um ihn in die gewünschte Form zu bringen. Dies geschieht in der Regel durch den Orthopädietechniker, der die orthopädietechnische Einrichtung herstellt. Dabei wird im einfachsten Fall der aus Metall bestehende Gelenkschenkel im kalten Zustand gebogen. Dadurch wird zwar die gewünschte Form erreicht, das Material jedoch mechanisch stark belastet, so dass seine Stabilität reduziert wird.

Um dies auszugleichen wird das Verbindungselement zunächst meist mit einer größeren Materialstärke hergestellt, als dies für die auftretenden Belastungen eigentlich notwendig wäre. Dadurch wird der Materialaufwand und damit die Kosten und das Gewicht des Verbindungselementes erhöht und zudem die umformende Bearbeitung durch den Orthopädietechniker erschwert.

Der Erfindung liegt daher die Aufgabe zugrunde, ein Verfahren zum Herstellen eines Verbindungselementes und einer orthopädietechnischen Einrichtung so weiterzuentwickeln, dass die Nachteile aus dem Stand der Technik behoben oder zumindest abgemildert werden.

Die Erfindung löst die gestellte Aufgabe durch ein Verfahren zum Herstellen eines Verbindungselementes zum Verbinden zweier Bauteile einer orthopädietechnischen Einrichtung für ein Körperteil, das die folgenden Schritte aufweist:
- Erfassen von 3-dimensionalen Scan-Daten zumindest eines Teils des Körperteils mittels eines Scanners,
- Bestimmen einer Soll-Position und/oder eine Soll-Orientierung des Verbindungselementes relativ zu dem Körperteil aus den Scan-Daten,
- Modellieren des Verbindungselementes mittels der Scan-Daten, der Soll-Position und/oder der Soll-Orientierung und Informationen über die zu verbindenden Bauteile und
- Fertigen des modellierten Verbindungselementes.

Durch das erfindungsgemäße Verfahren wird ein Verbindungselement hergestellt, das vorzugsweise nicht mehr durch den Orthopädietechniker umgeformt werden muss. Dadurch kann die Materialstärke auf das notwendige Mindestmaß reduziert werden, wodurch der Materialeinsatz und damit die Herstellungskosten gesenkt werden. Zudem verkürzt sich die Herstellungszeit für die orthopädietechnische Einrichtung, da der Orthopädietechniker weniger Verfahrensschritte durchführen muss.

Zunächst wird zumindest ein Teil des Körperteiles, vorzugsweise jedoch das gesamte Körperteil, mittels eines Scanners gescannt, wodurch die 3-dimensionalen Scan-Daten erfasst werden. Diese liegen in Form von elektronischen Daten vor, die beispielsweise in einem Datenspeicher einer elektronischen Datenverarbeitungseinrichtung hinterlegt werden können. Der Scanner ist vorzugsweise ein mobiles Gerät, das beispielsweise ein Handscanner ist. Ein solcher Handscanner ist vorzugsweise mit einer Hand bedienbar und wird von einer das Verfahren durchführenden Person so auf das Körperteil gerichtet, dass die Scan-Daten erfasst werden. Dabei wird das Körperteil vorzugweise mit einer geeigneten Scan-Strahlung bestrahlt, die anschließend mittels eines geeigneten Detektors detektiert wird. Die Scan-Strahlung ist beispielsweise ein Lichtmuster aus sichtbarem Licht und/oder Infrarotstrahlung und der Detektor eine für die verwendete Wellenlänge der Strahlung ausgelegte Kamera. Die Scan-Daten werden bevorzugt durch ein photogrammetrisches Verfahren erfasst. Mit anderen Worten wird zum Erfassen der Scan-Daten eine Photogrammetrie durchgeführt.

Alternativ oder zusätzlich dazu kann auch wenigstens ein stationärer Scanner verwendet werden. Auch in diesem Fall wird das zu scannende Körperteil vorzugsweise mit einer geeigneten Scan-Strahlung bestrahlt. Um das Körperteil herum sind eine Mehrzahl von Detektoren, beispielsweise geeignete Kameras angeordnet, die das Körperteil aus unterschiedlichen Raumrichtungen erfassen und abbilden. Mittels Bildbearbeitung werden aus den so erfassten Einzelbilden die Scan-Daten ermittelt. Die Prozesse und Verfahren zum Erfassen der Scan-Daten sind dabei zu den Prozessen und Verfahren sehr ähnlich oder gar identisch, die bei einem mobilen Scanner verwendet werden. Bei einem stationäre Scanner ist es jedoch möglich die Mehrzahl von Einzelbildern mit der Mehrzahl an Detektoren, insbesondere Kameras, gleichzeitig oder in zeitlich kurzen Abständen aufnehmen, während bei der Verwendung eines mobilen Scanner eine längere Zeit vergeht, bis die notwenigen Daten aus den unterschiedlichen Blickwinkeln und Perspektiven erfasst sind. Da während des Erfassens der Scan-Daten das Körperteil vorzugsweise nicht bewegt werden sollte, ist die Verwendung eines stationären Scanners von Vorteil. Allerdings erhöht sich dadurch der apparative Aufwand.

Nach dem Erfassen der Scan-Daten wird die Soll-Position und/oder die Soll-Orientierung des Verbindungselementes relativ zu dem Körperteil bestimmt. Anschließend wird das Verbindungselement modelliert, wobei die so bestimmte Soll-Position und/oder Soll-Orientierung berücksichtigt wird. Dadurch wird das Verbindungselement in einer geometrischen Ausgestaltung modelliert, die eine weitere Umformung zu einem späteren Zeitpunkt unnötig macht. Insbesondere wird der für das Verbindungselement zur Verfügung stehende Raum berücksichtigt, der sich aus den Scan-Daten, also den zugrunde liegenden körperlichen Gegebenheiten, und den zu verbindenden Bauteilen ergibt.

Vorzugsweise wird aus den Scan-Daten zunächst eine sogenannte Zweckform erstellt. Dazu werden die Scan-Daten derart modifiziert, dass eine an diese modifizierten Scan-Daten angepasste orthopädietechnische Einrichtung ihren Zweck am eigentlichen Körperteil gut, möglichst optimal erfüllt. Dies erfolgt bevorzugt automatisch mittels einer elektronischen Steuerung, beispielsweise einer innerhalb einer elektronischen Datenverarbeitungseinrichtung ablaufenden Software, die eingerichtet ist, die Scan-Daten zu modifizieren und so die Zweckform zu erstellen. Die Bestimmung der Soll-Position und/oder der Soll-Orientierung erfolgt in diesem Fall danach anhand der erstellten Zweckform. Beim Erstellen der Zweckform wird beispielsweise das Volumen eines Körperteils geändert, vorzugsweise vergrößert, oder eine Stellung und/oder Orientierung eines Körperteiles zu einem anderen Körperteil angepasst. So kann beispielsweise die Fußstellung relativ zum Unterschenkel angepasst und gegenüber der tatsächlichen Stellung, die durch die Scan-Daten abgebildet wird, verändert werden.

In einer bevorzugten Ausgestaltung des Verfahrens wird vor dem Erfassen der Scan-Daten wenigstens eine für den Scanner erkennbare Markierung auf das Körperteil aufgebracht. Die Markierung wird bevorzugt auf das Körperteil aufgeklebt oder mit einer mittels des Scanners erfassbaren Farbe auf das Körperteil aufgebracht. Wichtig ist, dass die Markierung auch in den Scan-Daten enthalten und erkennbar ist.

Durch die wenigstens eine Markierung werden vorzugsweise Stellen des Körperteils markiert, die für die Positionierung des Verbindungselementes und/oder eines oder beider Bauteile, die durch das Verbindungselement zu verbinden sind, wichtig sind. Diese Stellen markieren bevorzugt den Verlauf und/oder die Lage von Gelenkachsen des Körperteiles, besonders empfindliche Stellen wie etwa empfindliche Weichteile, Narbengewebe oder Stellen, bei denen ein Knochen oder Knochenende nur von einer dünnen Schicht Gewebe überdeckt ist. Werden mehrere unterschiedliche Stellen des Körperteiles mit jeweils einer Markierung versehen, werden bevorzugt verschiedene Markierungen verwendet. Damit ist es bevorzugt möglich, aus der Art der Markierung in den Scan-Daten die einzelnen markierten Stellen zu identifizieren.

Vorzugsweise weist eines der zu verbindenden Bauteile und/oder das Verbindungselement ein Gelenk auf. Bevorzugt wird die Soll-Position und/oder die Soll-Orientierung so bestimmt, dass eine Gelenkachse des Gelenks mit einer Gelenkachse des Körperteils übereinstimmt. Dies ist besonders einfach möglich, wenn die Gelenkachse des Körperteiles auf dem Körperteil mit einer Markierung versehen wurde und diese in den Scan-Daten erkennbar ist.

In einer bevorzugten Ausgestaltung werden die Scan-Daten in zwei unterschiedlichen Positionen und/oder Belastungen des Körperteils erfasst. Dies ist insbesondere dann von Vorteil, wenn die orthopädietechnische Einrichtung eine Kraft auf das Körperteil aufbringen soll, die zu einer Verformung des Körperteils führt. Bevorzugt wird die orthopädietechnische Einrichtung an das Körperteil angelegt, ohne dass zunächst eine Kraft aufgebracht wird. Für diese Situation werden bevorzugt die Scan-Daten, die im unbelasteten Zustand des Körperteils erfasst wurden, verwendet. Erst nach dem Anlegen wird die orthopädietechnische Einrichtung die gewünschte Kraft aufbringen und die Verformung des Körperteils hervorrufen. Dazu kann die orthopädietechnische Einrichtung angepasst werden, indem beispielsweise ein Gurt gespannt oder ein Gelenk eingestellt wird. Alternativ oder zusätzlich dazu kommt es zu der Verformung durch eine Belastung des Körperteils mit der sich daran befindenden orthopädietechnischen Einrichtung. Dies ist insbesondere relevant bei orthopädietechnischen Einrichtungen in Form von Prothesenschäften, insbesondere für die untere Extremität.

Bevorzugt wird beim Modellieren des Verbindungselementes eine Simulation der erwarteten Belastung durchgeführt, deren Ergebnis in das Modellieren des Verbindungselementes einfließt. Es werden bevorzugt Bewegungen des Körperteils, an dem die orthopädietechnische Einrichtung angeordnet wird, simuliert und die dabei auftretenden Kräfte ermittelt. Das Verbindungselement wird dann so modelliert, dass es den ermittelten Kräften standhält. Dadurch werden die geometrische Form und Materialstärke des Verbindungselementes optimiert, ohne dass die Betriebssicherheit des Verbindungselementes eingeschränkt wird. Insbesondere werden Materialverjüngungen und/oder Durchbrüche an den Stellen in das Modell des Verbindungselement integriert, an denen die Simulation der erwarteten Belastungen eine geringe Belastung erwarten lässt, die dies erlaubt.

Bevorzugt wird bei der Modellierung des Verbindungselement auch eine Soll-Position und/oder Soll-Orientierung relativ zu wenigstens einem der beiden Bauteile, bevorzugt relativ zu beiden Bauteilen bestimmt. Dazu kann eine Position und/oder Orientierung des Verbindungselementes variiert werden, wobei bevorzugt die erwarteten Belastungen und Kräfte für jede neue Position ermittelt werden.

Vorzugsweise wird das Verbindungselement mittels eines additiven Fertigungsverfahrens, insbesondere eines 3D-Druckverfahrens hergestellt. Dabei wird bevorzugt als Druckmaterial ein Metall und/oder ein Kunststoff, insbesondere ein Duroplast oder Polymer verwendet. Besonders bevorzugt werden Fasern als stabilisierende Elemente in das Druckmaterial eingebracht. Diese können textile Fasern, Naturfasern, Kunstfasern, Glasfasern, Kohlefasern oder Metallfasern sein. Unter einer Faser wird dabei insbesondere ein nahezu eindimensionales Element verstanden, das folglich in einer ersten Richtung, die als Längsrichtung bezeichnet wird, eine deutlich größere Ausdehnung aufweist, als in der senkrecht auf dieser Längsrichtung stehenden Ebene.

Bevorzugt werden die Fasern kontrolliert während des additiven Fertigungsverfahrens in das Druckmaterial eingebracht. Dies bedeutet, dass vorzugsweise bereits bei der Modellierung des Verbindungselementes die Menge und Richtung der Längserstreckung der Fasern vorbestimmt wird. Besonders bevorzugt wird auch die Länge der einzubringenden Fasern vorbestimmt.

Während der Modellierung des Verbindungselementes werden die Menge, die Länge, das Material und/oder die Längsrichtung der einzubringenden Fasern berücksichtigt. Dies ermöglicht es, die Stabilität des Verbindungselementes in vorbestimmten Bereichen und in vorbestimmte Richtungen zu beeinflussen und so die benötigte Materialmenge weiter zu reduzieren.

Die Erfindung löst die gestellte Aufgabe zudem durch ein Verfahren zum Herstellen einer orthopädietechnischen Einrichtung mit wenigstens zwei Bauteilen, die zumindest auch durch wenigstens ein Verbindungselement verbunden sind, wobei das Verfahren folgende Schritte aufweist:
- Herstellen des Verbindungselementes nach einem der hier beschriebenen Verfahren und
- Verbinden der beiden Bauteile mittels des hergestellten Verbindungselementes.

In einer bevorzugten Ausgestaltung bildet das Verbindungselement mit einem der zu verbindenden Bauteile ein Gelenk, durch das die beiden Bauteile im verbundenen Zustand relativ zueinander bewegbar, vorzugsweise verschwenkbar sind. Dazu ist es beispielsweise von Vorteil, wenn das Verbindungselement und eines der Bauteile jeweils eine Bohrung aufweisen. Diese Bohrungen werden bei der Montage der orthopädietechnischen Einrichtung in Überdeckung miteinander gebracht. Dann wird ein Achselement, beispielsweise eine Schraube, ein Stift, Stab oder Bolzen durch die beiden Bohrungen geführt und darin befestigt, so dass das Verbindungselement relativ zu dem Bauteil um dieses Achselement schwenkbar ist. Wird das Verbindungselement an dem anderen der beiden Bauteile starr, insbesondere drehfest angeordnet, sind die beiden Bauteile, die durch das Verbindungselement verbunden sind, relativ zueinander verschwenkbar. Vorzugsweise verfügt das Verbindungselement über wenigstens eine Befestigungsschiene, besonders bevorzugt zwei Befestigungsschienen, die an den beiden Gelenkteilen des Gelenkes, die relativ zueinander bewegbar sind, angeordnet sind. Besonders bevorzugt ist die wenigstens eine Befestigungsschiene einstückig mit dem jeweiligen Gelenkteil ausgebildet.

Vorzugsweise weisen die beiden Bauteile jeweils wenigstens einen Abschnitt auf, die direkt, vorzugsweise gelenkig, besonders bevorzugt mittels eines Mitläufergelenks aneinander angeordnet sind. Auf diese Weise wird beispielsweise ein Mitläufergelenk hergestellt. Bei einer orthopädietechnischen Einrichtung, bei der das Verbindungselement auf einer Seite des Körperteils, beispielsweise lateral, angeordnet ist, sind die beiden Abschnitte bevorzugt auf der gegenüberliegenden Seite, beispielsweise medial, angeordnet. Vorzugsweise sind die Abschnitte so angeordnet, dass sie im verbundenen Zustand Torsionskräfte reduzieren, das Verbindungselement entlasten und/oder die Stabilität der orthopädietechnischen Einrichtung und/oder ihrer Befestigung am Körperteil verbessern.

Besonders bevorzugt wird ein Befestigungsabschnitt wenigstens eines der beiden Bauteile nach dem Modellieren des Verbindungselementes oder bei Modellieren des Verbindungselementes mittels der Scan-Daten, der Soll-Position und/oder der Soll-Orientierung und Informationen über das jeweilige Bauteil modelliert. Besonders bevorzugt wird eines der beiden Bauteile, besonders bevorzugt werden beide Bauteile, nach dem Modellieren des Verbindungselementes oder bei Modellieren des Verbindungselementes mittels der Scan-Daten, der Soll-Position und/oder der Soll-Orientierung und Informationen über das jeweilige Bauteil modelliert.

In einer bevorzugten Ausgestaltung des Verfahrens ist wenigstens eines der beiden Bauteile der herzustellenden orthopädietechnischen Einrichtung ein Faserverbundbauteil. Besonders bevorzugt sind beide zu verbindenden Bauteile Faserverbundbauteile. Dies können Kohlefaser-Verbundbauteile oder Glasfaser-Verbundbauteile oder andere Verbundbauteile sein. In diesem Fall ist es von Vorteil, wenn das Verbindungselement beim Verbinden mit dem wenigstens einen als Verbundbauteil ausgebildeten Bauteil, bevorzugt jedoch mit beiden als Verbundbauteile ausgebildeten Bauteilen verbunden wird und dabei in den Lagenaufbau des jeweiligen Bauteiles integriert wird. Faserverbund-Bauteile werden aus sogenannten Prepregs hergestellt, bei denen es sich in der Regel um Gewebeelemente, beispielsweise Fasermatten aus dem jeweiligen Fasermaterial handelt. Diese werden in die gewünschte Form eingelegt und in der Regel mit einem Kunstharz beschichtet, das beim Aushärten den Faserverbundwerkstoff hergestellt. Oftmals sind auch die einzelnen Fasermatten bereits mit dem Kunstharz getränkt oder beschichtet.

In diesem Zustand der Bauteile wird das Verbindungselement ebenfalls in der Form, insbesondere zwischen verschiedenen Lagen der Fasermatten des Verbundmaterials angeordnet.

Mithilfe der beigefügten Zeichnungen werden nachfolgend einige Ausführungsbeispiele der vorliegenden Erfindung näher erläutert. Es zeigt:
- Figur 1-: eine Knöchelorthese in einer Rückansicht,
- Figur 2-: die Knöchelorthese aus Figur 1 in einer Seitenansicht,
- Figur 3-: eine weitere Knöchelorthese in einer Seitenansicht,
- Figur 4-: die Knöchelorthese aus Figur 3 in einer Rückansicht
- Figur5 -: eine weitere Knöchelorthese in einer Rückansicht und
- Figur 6a - 6e: schematische Darstellungen verschiedener Verfahrensschritte.

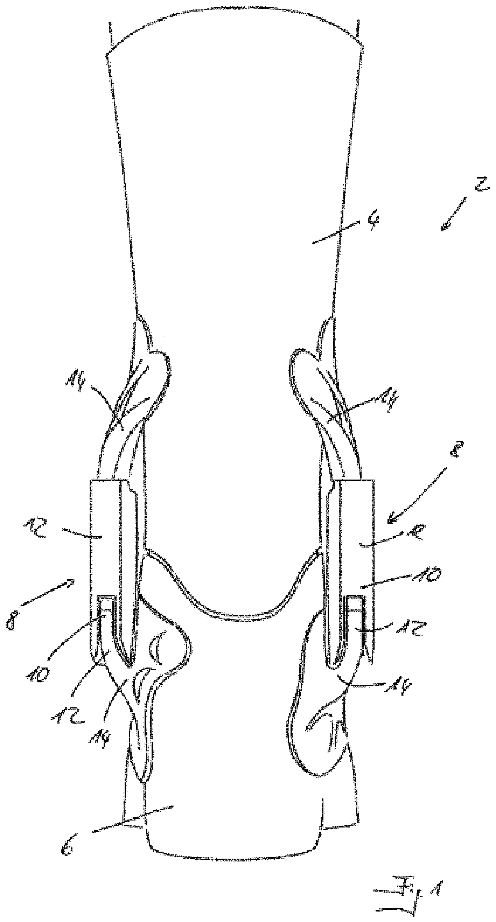

Figur 1 zeigt eine orthopädietechnische Einrichtung 2 in Form einer Knöchelorthese. Sie verfügt über ein erstes Bauteil 4 und ein zweites Bauteil 6, wobei im vorliegenden Ausführungsbeispiel das erste Bauteil 4 an einem nicht dargestellten Unterschenkel eines Benutzers und das zweite Bauteil 6 an einem nicht dargestellten Fuß des Benutzers angeordnet wird. Das erste Bauteil 4 und das zweite Bauteil 6 sind durch zwei Verbindungselemente 8 miteinander verbunden, von denen im gezeigten Ausführungsbeispiel jedes ein Gelenk 10 mit zwei Gelenkteilen 12 aufweist, die gegeneinander verschwenkbar sind.

Die Gelenkteile 12 sind jeweils mit einer Befestigungsschiene 14 verbunden oder einstückig mit einer solchen Befestigungsschiene 14 ausgebildet. Die Verbindungselemente 8 sind individuell an die Form und Orientierung des Knöchels, des Fußes und/oder des Unterschenkels des Benutzers der orthopädietechnischen Einrichtung 2 angepasst. Dazu sind im gezeigten Ausführungsbeispiel die Befestigungsschienen 14 individuell ausgebildet. Man erkennt lateral und medial unterschiedlich ausgebildete Befestigungsschienen, die den unterschiedlichen Geometrien und unterschiedlichen erwarteten Belastungen Rechnung tragen.

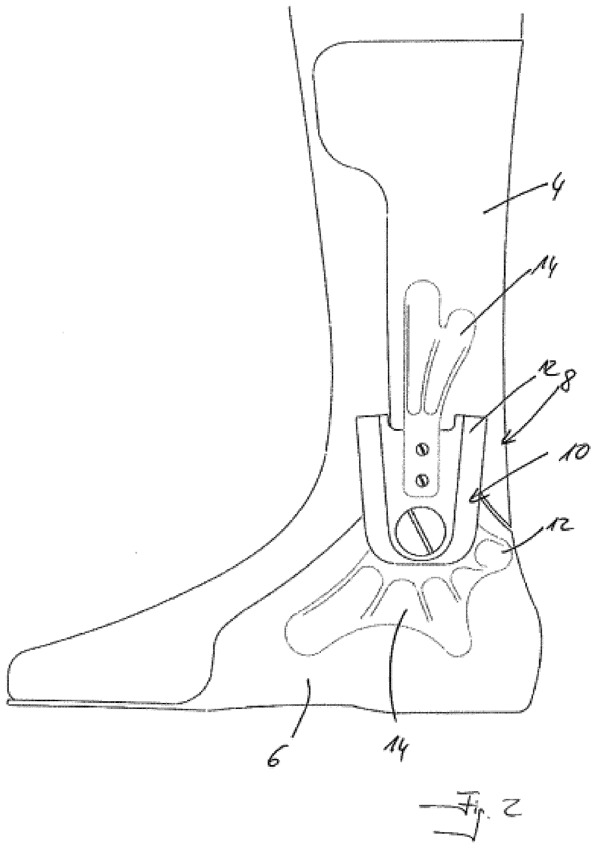

Figur 2 zeigt die orthopädietechnische Einrichtung 2 aus Figur 1 in einer Seitenansicht. Das erste Bauteil 4 und das zweite Bauteil 6 sind über das Verbindungselement 8 mit Gelenk 10 und den beiden Gelenkteilen 12 miteinander verbunden. Am proximalen, also in Figur 2 oberen, Gelenkteil 12 die Befestigungsschiene 14 angeschraubt ist, ist die Befestigungsschiene 14 am distalen Gelenkteil 12 einstückig angeordnet.

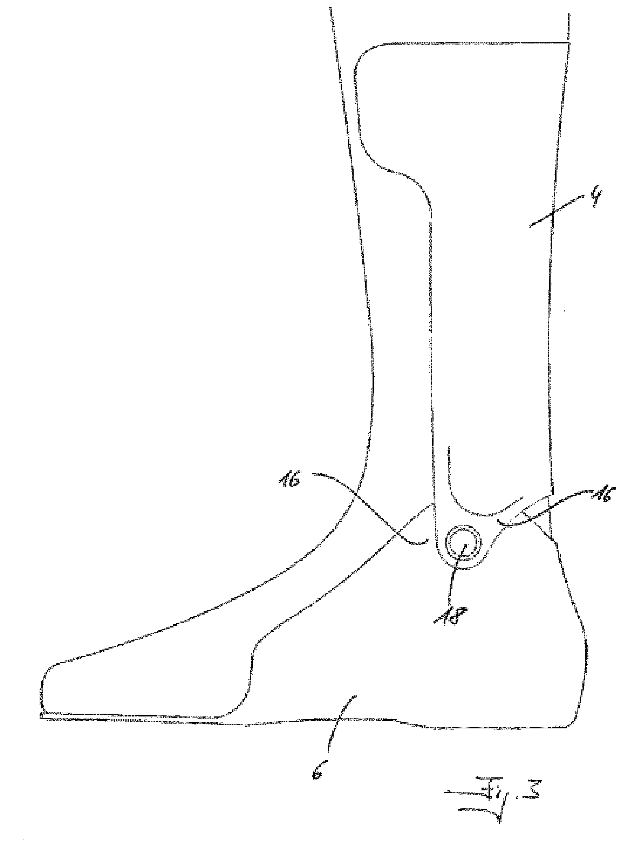

Figur 3 zeigt eine weitere orthopädietechnische Einrichtung 2 in einer Seitenansicht. Das erste Bauteil 4 und das zweite Bauteil 6 verfügen über jeweils einen Abschnitt 16, die direkt miteinander verbunden sind und ein Mitläufergelenk 18

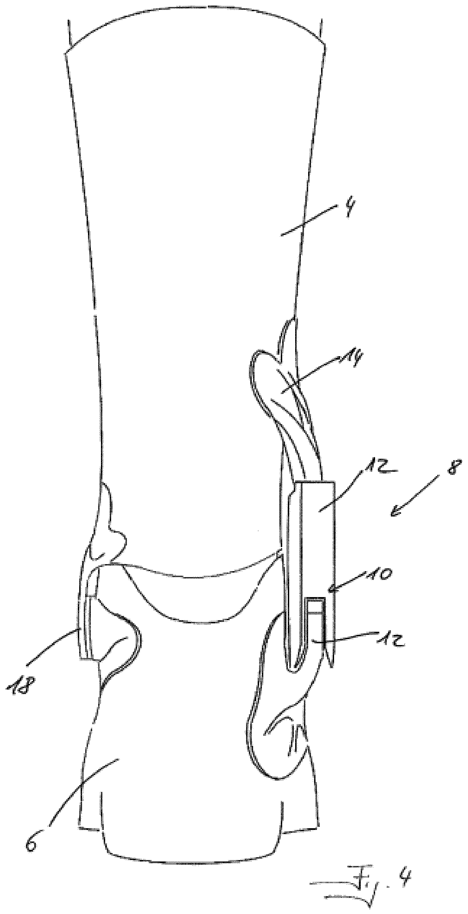

Figur 4 zeigt die orthopädietechnische Einrichtung 2 aus Figur 3 in der Rückansicht. Das erste Bauteil 4 und das zweite Bauteil 2 sind, wie bereits in Figur 3 dargestellt, durch das Mitläufergelenk 18 an einer Seite, vorliegend medial, direkt miteinander verbunden, während auf der gegenüberliegenden Seite, vorliegend lateral, ein Verbindungselement 8 angeordnet ist. Dies entspricht dem Verbindungselement 8 aus Figur 1. Es verfügt über das Gelenk 10 mit den beiden Gelenkteilen 12 an denen die jeweiligen Befestigungsschienen 14 angeordnet sind.

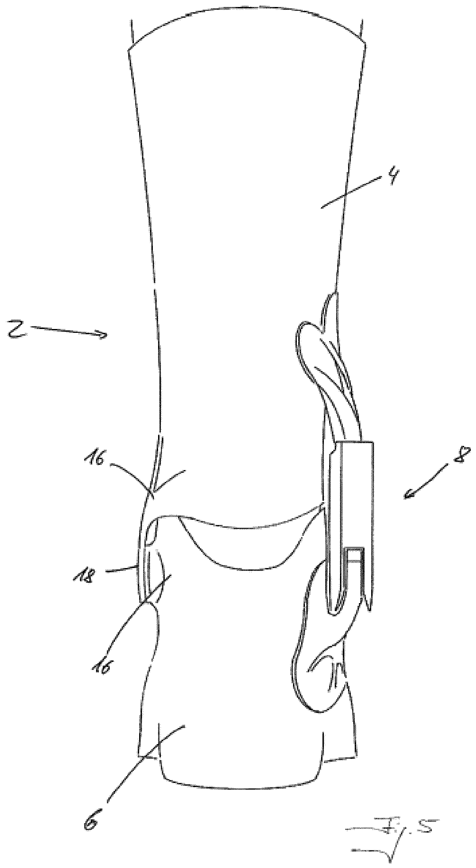

Figur 5 zeigt eine orthopädietechnische Einrichtung 2, die der in Figur 4 gezeigten Einrichtung entspricht. Sie unterscheiden sich durch eine unterschiedliche Ausgestaltung der Abschnitte 16 an denen der beiden Bauteile 4, 6 direkt miteinander verbunden sind und das Mitläufergelenk 18 bilden.

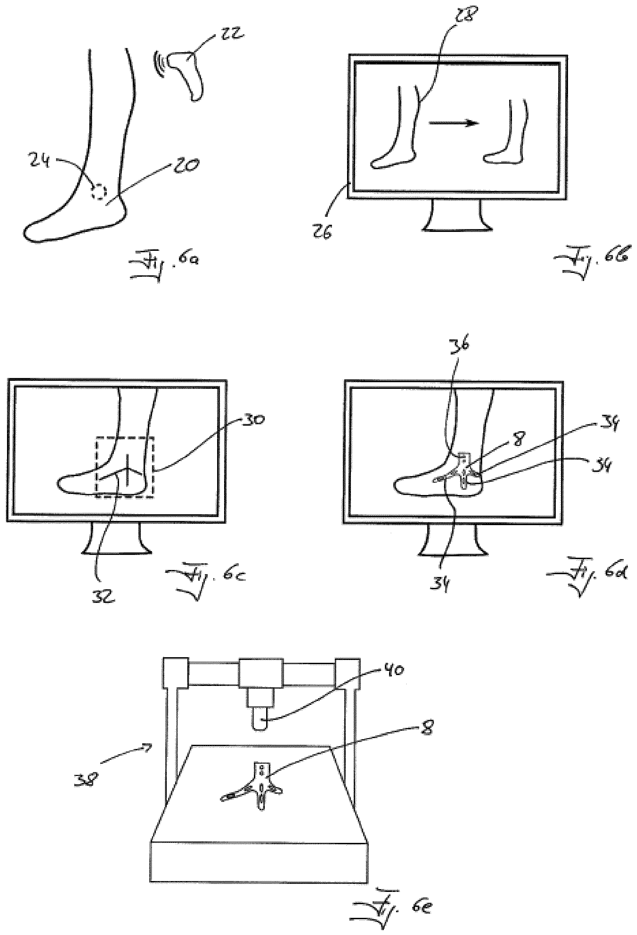

In den Figuren 6a bis 6e sind verschiedene Verfahrensschritte dargestellt. Figur 6a zeigt schematisch einen Fuß 20, der mit einem Scanner 22 vermessen und gescannt wird. Dabei werden 3-dimensionale Scan-Daten von dem Fuß 20, der in diesem Fall das zu Scannen der Körperteil darstellt, erfasst. Auf dem Fuß 20 ist eine Markierung 24 aufgebracht, die die Position einer Schwenkachse, in diesem Fall einer Knöchelachse, darstellt. Die Markierung 24 ist so gewählt, dass sie vom Scanner 22 erfasst werden kann und somit Teil der 3-dimensionalen Scan-Daten ist.

In Figur 6b ist ein Monitor 26 dargestellt. Damit wird illustriert, dass dieser und die Verfahrensschritte der folgenden beiden Abbildungen mittels einer elektronischen Datenverarbeitungseinrichtung, insbesondere eines Computers, ausgeführt werden. Im linken Teil des Monitors 26 sind die 3-dimensionalen Scan-Daten 28 gezeigt, die im gezeigten Ausführungsbeispiel den Fuß 20 in genau der Position zeigen, in der er durch den Scanner 22 erfasst wurde. In diesem Verfahrensschritt wird eine Soll-Position des Fußes 20 erstellt, in der der Fuß durch die herzustellende orthopädietechnische Einrichtung 2, die im gezeigten Ausführungsbeispiel eine Fußheberorthese sein kann, gehalten werden soll. Man erkennt, dass der Vorfußbereich angehoben wird.

In Figur 6c ist dargestellt, dass an die so modulierten Soll-Daten ein Verbindungselement schematisch angepasst wird. Dabei wird eine Soll-Position und/oder eine Soll-Orientierung des Verbindungselementes 8 relativ zu dem 20 ermittelt. Dies ist durch den gestrichelten Kasten 30 und das darin gezeigte Achsenkreuz 32 dargestellt. In diesem Verfahrensschritt geht es im Wesentlichen darum, die Position und Orientierung festzustellen. Individuelle Ausgestaltungen der einzelnen Elemente oder Bauteile des Verbindungselementes 8 werden in diesem Schritt noch nicht bestimmt.

Dies geschieht im in Figur 6d gezeigten Verfahrensschritt. Das Verbindungselement 8 wird an die Soll-Daten des Fußes 20 an modelliert. Es verfügt im gezeigten Ausführungsbeispiel über drei nach unten ragende Arme 34, die Befestigungsabschnitte bilden und an denen das Verbindungselement 8 später mit dem zweiten Bauteil 6, Fall einem Fußteil, verbunden wird. Das Verbindungselement 8 verfügt zudem über eine Befestigungseinrichtung 36, die ebenfalls einen Befestigungsabschnitt bildet und mit der es im gezeigten Ausführungsbeispiel an einem ersten Bauteil 4, vorliegend an einer Unterschenkelschiene, befestigbar ist.

In Figur 6e ist schematisch dargestellt, wie nach den so modulierten Daten das Verbindungselement 8 hergestellt wird. Dazu wird im gezeigten Ausführungsbeispiel ein 3-D-Drucker 38 mit wenigstens einem Druckkopf 40 verwendet.

### Bezugszeichenliste

- 2: orthopädietechnische Einrichtung
- 4: erstes Bauteil
- 6: zweites Bauteil
- 8: Verbindungselement
- 10: Gelenk
- 12: Gelenksteil
- 14: Befestigungsschiene
- 16: Abschnitt
- 18: Mitläufergelenk
- 20: Fuß
- 22: Scanner
- 24: Markierung
- 26: Monitor
- 28: Scan-Daten
- 30: gestrichelter Kasten
- 32: Achsenkreuz
- 34: Arm
- 36: Befestigungseinrichtung
- 38: 3-D-Drucker
- 40: Druckkopf

## Patentansprüche

1. Verfahren zum Herstellen eines Verbindungselementes (8) zum Verbinden zweier Bauteile (4, 6) einer orthopädietechnischen Einrichtung (2) für ein Körperteil, wobei das Verfahren die folgenden Schritte aufweist:
• Erfassen von 3-dimensionalen Scan-Daten (28) zumindest eines Teils des Körperteils mittels eines Scanners (22),
• Bestimmen einer Soll-Position und/oder einer Soll-Orientierung des Verbindungselementes (8) relativ zu dem Körperteil aus den Scan-Daten (28),
• Modellieren des Verbindungselementes (8) mittels der Scan-Daten (28), der Soll-Position und/oder der Soll-Orientierung und Informationen über die zu verbindenden Bauteile (4, 6) und
• Fertigen des modellierten Verbindungselementes (8).

2. Verfahren nach Anspruch 1, **dadurch gekennzeichnet, dass** vor dem Erfassen der Scan-Daten (28) wenigstens eine für den Scanner (22) erkennbare Markierung (24) auf das Körperteil aufgebracht wird.

3. Verfahren nach Anspruch1 oder 2, **dadurch gekennzeichnet, dass** wenigstens eines der zu verbindenden Bauteile (4, 6) und/oder das Verbindungselement (8) ein Gelenk (10) aufweist.

4. Verfahren nach einem der vorstehenden Ansprüche, **dadurch gekennzeichnet, dass** die Scan-Daten (28) in wenigstens zwei unterschiedlichen Positionen und/oder Belastungen des Körperteils erfasst werden.

5. Verfahren nach einem der vorstehenden Ansprüche, **dadurch gekennzeichnet, dass** beim Modellieren des Verbindungselementes (8) eine Simulation der erwarteten Belastung durchgeführt wird, deren Ergebnis in das Modellieren des Verbindungselementes (8) einfließt.

6. Verfahren nach einem der vorstehenden Ansprüche, **dadurch gekennzeichnet, dass** das modellierte Verbindungselement (8) mittels eines additiven Fertigungsverfahrens, insbesondere eines 3D-Druckverfahrens, hergestellt wird.

7. Verfahren zum Herstellen einer orthopädietechnischen Einrichtung (2) mit wenigstens zwei Bauteilen (4, 6), die zumindest auch durch ein Verbindungselement (8) verbunden sind, wobei das Verfahren die folgenden Schritte aufweist:
• Herstellen des Verbindungselementes (8) nach einem der vorstehenden Ansprüche, und
• Verbinden der beiden Bauteile (4, 6) mittels des hergestellten Verbindungselementes (8).

8. Verfahren nach Anspruch 7, **dadurch gekennzeichnet, dass** die beiden Bauteile (4, 6) jeweils wenigstens einen Abschnitt (16) aufweisen, die direkt, vorzugsweise gelenkig, besonders bevorzugt mittels eines Mitläufergelenkes (18), aneinander angeordnet sind.

9. Verfahren nach Anspruch 7 oder 8, **dadurch gekennzeichnet, dass** ein Befestigungsabschnitt (34, 36) wenigstens eines der beiden Bauteile (4, 6) nach dem Modellieren des Verbindungselementes (8) oder beim Modellieren des Verbindungselementes (8) mittels der Scan-Daten (28), der Soll-Position und/oder der Soll-Orientierung und Informationen über das jeweilige Bauteil (4, 6) modelliert wird.

10. Verfahren nach einem der Ansprüche 7 bis 9, **dadurch gekennzeichnet, dass** wenigstens eines der beiden Bauteile (4, 6) ein Faserverbundbauteil ist und das Verbindungselement (8) beim Verbinden mit diesem wenigstens einen Bauteil (4, 6) in einen Lagenaufbau des Bauteiles (4, 6) integriert wird.

## Claims

1. A method for producing a connecting element (8) for connecting two components (4, 6) of an orthopaedic device (2) for a body part, the method comprising the following steps:
• capturing 3-dimensional scan data (28) of at least one part of the body part by means of a scanner (22),
• determining a target position and/or a target orientation of the connecting element (8) relative to the body part from the scan data (28),
• modelling the connecting element (8) using the scan data (28), the target position and/or the target orientation and information on the components (4, 6) to be connected, and
• manufacturing the modelled connecting element (8).

2. The method according to claim 1, **characterised in that**, prior to capturing the scan data (28), at least one marker (24) that can be recognised by the scanner (22) is applied to the body part.

3. The method according to claim 1 or 2, **characterised in that** at least one of the components (4, 6) to be connected and/or the connecting element (8) has a joint (10).

4. The method according to one of the preceding claims, **characterised in that** the scan data (28) is captured in at least two different positions and/or loads of the body part.

5. The method according to one of the preceding claims, **characterised in that**, during modelling of the connecting element (8), a simulation of the anticipated load is performed, the result of which is included in the modelling of the connecting element (8).

6. The method according to one of the preceding claims, **characterised in that** the modelled connecting element (8) is produced by means of an additive manufacturing process, especially a 3D printing process.

7. A method for producing an orthopaedic device (2) with at least two components (4, 6), which are at least also connected by a connecting element (8), the method comprising the following steps:
• producing the connecting element (8) according to one of the preceding claims and
• connecting the two components (4, 6) by means of the connecting element (8) produced.

8. The method according to claim 7, **characterised in that** the two components (4, 6) each comprise at least one section (16) which are arranged directly, preferably in an articulated manner, particularly preferably by means of a support joint (18).

9. The method according to claim 7 or 8, **characterised in that** a fastening section (34, 36) of at least one of the two components (4, 6) is modelled after modelling of the connecting element (8) or during modelling of the connecting element (8) by means of the scan data (28), the target position and/or the target orientation and information on the respective component (4, 6).

10. The method according to one of the claims 7 to 9, **characterised in that** at least one of the two components (4, 6) is a fibre-composite component and the connecting element (8) is integrated into a layer structure of the component (4, 6) when being connected to this at least one component (4, 6).

## Revendications

1. Procédé de fabrication d'un élément de liaison (8) destiné à relier deux composants (4, 6) d'un dispositif orthopédique (2) pour une partie du corps, le procédé comprenant les étapes suivantes consistant à :
• acquérir des données de balayage tridimensionnelles (28) d'au moins une portion de la partie du corps au moyen d'un scanner (22),
• déterminer une position de consigne et/ou une orientation de consigne de l'élément de liaison (8) par rapport à la partie du corps à partir des données de balayage (28),
• modéliser l'élément de liaison (8) au moyen des données de balayage (28), de la position de consigne et/ou de l'orientation de consigne et d'informations relatives aux composants (4, 6) à relier, et
• fabriquer l'élément de liaison (8) modélisé.

2. Procédé selon la revendication 1,
**caractérisé en ce qu'**au moins un marquage (24) reconnaissable par le scanner (22) est appliqué sur la partie du corps avant l'acquisition des données de balayage (28).

3. Procédé selon la revendication 1 ou 2,
**caractérisé en ce que** l'un au moins des composants (4, 6) à relier et/ou l'élément de liaison (8) comprend une articulation (10).

4. Procédé selon l'une des revendications précédentes,
**caractérisé en ce que** les données de balayage (28) sont acquises selon au moins deux positions et/ou contraintes différentes de la partie du corps.

5. Procédé selon l'une des revendications précédentes,
**caractérisé en ce que**, lors de la modélisation de l'élément de liaison (8), on effectue une simulation de la contrainte attendue, dont le résultat est pris en compte dans la modélisation de l'élément de liaison (8).

6. Procédé selon l'une des revendications précédentes,
**caractérisé en ce que** l'élément de liaison (8) modélisé est fabriqué par un procédé de fabrication additive, en particulier par un procédé d'impression 3D.

7. Procédé de fabrication d'un dispositif orthopédique (2) comprenant au moins deux composants (4, 6) qui sont reliés au moins également par un élément de liaison (8), le procédé comprenant les étapes suivantes consistant à :
• fabriquer l'élément de liaison (8) selon l'une des revendications précédentes, et
• relier les deux composants (4, 6) au moyen de l'élément de liaison (8) fabriqué.

8. Procédé selon la revendication 7,
**caractérisé en ce que** les deux composants (4, 6) comportent chacun au moins une portion (16), lesdites portions étant agencées directement l'une sur l'autre, de préférence de manière articulée, de manière particulièrement préférée au moyen d'une articulation suiveuse (18).

9. Procédé selon la revendication 7 ou 8,
**caractérisé en ce qu'**une portion de fixation (34, 36) de l'un au moins des deux composants (4, 6) est modélisée, après la modélisation de l'élément de liaison (8) ou lors de la modélisation de l'élément de liaison (8), au moyen des données de balayage (28), de la position de consigne et/ou de l'orientation de consigne et d'informations relatives au composant respectif (4,6).

10. Procédé selon l'une des revendications 7 à 9,
**caractérisé en ce que** l'un au moins des deux composants (4, 6) est un composant composite à fibres, et l'élément de liaison (8) est intégré dans une structure en couches du composant (4, 6) lors de la liaison avec ledit au moins un composant (4, 6).
